Europäisches Patentamt

European Patent Office

Office européen des brevets

(19)

(11) **EP 1 034 486 B1**

(12) **EUROPÄISCHE PATENTSCHRIFT**

(45) Veröffentlichungstag und Bekanntmachung des Hinweises auf die Patenterteilung:
**26.09.2001   Patentblatt 2001/39**

(21) Anmeldenummer: **98965090.8**

(22) Anmeldetag: **21.11.1998**

(51) Int Cl.7: **G06F 17/00**

(86) Internationale Anmeldenummer:
**PCT/DE98/03443**

(87) Internationale Veröffentlichungsnummer:
**WO 99/27463 (03.06.1999 Gazette 1999/22)**

(54) **VERFAHREN ZUR BESTIMMUNG WENIGSTENS EINER DIAGNOSTISCHEN INFORMATION AUS SIGNALMUSTERN MEDIZINISCHER SENSORSYSTEME**

METHOD FOR DETERMINING AT LEAST ONE DIAGNOSTIC PIECE OF INFORMATION FROM SIGNAL PATTERNS OF MEDICAL SENSOR SYSTEMS

PROCEDE PERMETTANT DE DETERMINER AU MOINS UNE INFORMATION DIAGNOSTIQUE A PARTIR DE MODELES DE SIGNAUX DE SYSTEMES DETECTEURS MEDICAUX

(84) Benannte Vertragsstaaten:
**BE CH DE ES FR GB IT LI NL**

(30) Priorität: **25.11.1997   DE 19752094**

(43) Veröffentlichungstag der Anmeldung:
**13.09.2000   Patentblatt 2000/37**

(73) Patentinhaber: **Bundesrepublik Deutschland, vertr. durch den Bundesminister für Wirtsch. vertr. durch den Präs. der Phys.-Techn. Bundesanstalt
38116 Braunschweig (DE)**

(72) Erfinder:
• **BOUSSELJOT, Ralf
  D-15711 Königs Wusterhausen (DE)**
• **KREISELER, Dieter
  D-10551 Berlin (DE)**

(74) Vertreter: **Lins, Edgar, Dipl.-Phys. Dr.jur. et al
Gramm, Lins & Partner GbR,
Theodor-Heuss-Strasse 1
38122 Braunschweig (DE)**

(56) Entgegenhaltungen:
EP-A- 0 642 760          WO-A-97/08989
DE-A- 19 638 738         US-A- 5 277 189

Anmerkung: Innerhalb von neun Monaten nach der Bekanntmachung des Hinweises auf die Erteilung des europäischen Patents  kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Auswertung von gemessenen periodischen oder quasiperiodischen Signalen medizinischer Sensorsysteme durch Digitalisierung der Signale und Vergleich von Signalabschnitten der gemessenen Signale mit abgespeicherten vergleichbaren Signalabschnitten.

Charakteristik bekannter technischer Lösungen:

Die in der zugänglichen Literatur beschriebenen Verfahren zur Feststellung der Signalmusterähnlichkeit von periodischen Signalen medizinischer Sensorsysteme beschränken sich in der Regel auf die Signale desselben Patienten.

Dabei ist bekannt, eine Korrelation von unter normalen Umständen gemessenen und gefilterten EKG-Signalen mit laufender, u.U. gestörten bzw. krankhaften EKG-Signalen desselben durchzuführen. Ein weiteres Beispiel ist US 5,240,009. Hier wird die Erkennung von Rhythmusstörungen durch Vergleich gemittelter und gespeicherter Wellenformkomplexe mit den aktuell gemessenen desselben Patienten beschrieben. Auch in DE 32 09 850 werden Rhythmusstörungen klassifiziert. Dies erfolgt durch Vergleich des vollständigen Verlaufs des EKG mit zuvor in einer Lernphase erfaßten oder berechneten EKG-Verläufen des untersuchten Patienten und der vollständigen Abspeicherung eines Beispiels des EKG-Verlaufs für jede Klasse von Rhythmusstörungen des untersuchten Patienten. Allen vorgestellten Lösungen ist gemeinsam, daß sie nur den Vergleich von EKG oder von Teilen des EKG am gleichen Patienten ermöglichen.

EKG-Auswertesysteme, z.B. nach US 5,022,404, erfassen ein oder mehrere Elektrodenpotentiale von am Patienten befestigten Elektroden, filtern und digitalisieren diese. Anschließend werden diese Signale über einen Multiplexer an einen im EKG-Auswertesystem vorhandenen Mikrocomputer mit CPU, Arbeitsspeicher usw. zugeführt. Dieser bereitet die gemessenen Signale auf, z.B. durch Entfernen der Grundliniendrift nach DE 4,106,856, US 5,357,969 oder die Entfernung von Muskelartefakten aus dem EKG nach US 5,259,387. Außerdem berechnet er die für die medizinische Bewertung des EKG erforderlichen medizinischen Ableitungen nach Wilson, Goldberger, Einthoven und/ oder die orthogonalen Ableitungen nach Frank. Im einfachsten Fall werden diese medizinischen Ableitungen entweder auf Papierstreifen und/oder elektronischen Displays, z.B. in US 5,022,404 auf LCD-Displays, dargestellt und vom auswertenden Arzt bewertet. Intelligentere, sogenannte auswertende Elektrokardiografen, verwenden den im Gerät vorhandenen Mikrocomputer außer zur Signalaufbereitung und Anzeige auch zur Signalauswertung, Signalvermessung und ggf. zur Ausgabe von diagnostischen Hinweisen, wie z.B. in US 5,029,082.

Die Signalvermessung und Auswertung erfolgt, wie noch in nachfolgend näher erläuterten Patentschriften beschrieben wird, in der Regel so, daß aus den berechneten medizinischen Ableitungen eine Anzahl, für die kardiologische Begutachtung des EKG wichtiger, einzelner Signalparameter hinsichtlich Zeitdauer und Amplitude bzw. daraus abgeleiteten Kriterien ermittelt werden. Problematisch bei dieser Ermittlung einzelner Signalkenngrößen sind die unterschiedlichen Herangehensweisen, wie z.B. bei der exakten Bestimmung der Nullinie des EKG /1/ zur Bestimmung des Anfangspunktes der P-Welle und der daraus folgenden Ermittlung der Dauer der P-Welle, die je nach Qualität des eingesetzten Verfahrens durchaus wesentlich abweichende Ergebnisse liefern. Die Patentschriften sind unter anderem DE 43 10 412 (Auswertung des ST-Segments bzw. der T-Welle), DE 39 27 709 (Auswertung der ST-Strecke), US 5,159,932 (Filterung des EKG, QRS-Findung, Mittelung) oder US 5,020,540 (Analyse der Frequenzstruktur des QRST-Komplexes, Waveform-Template). Weitere relevante Patentschriften beinhalten die Ermittlung einzelner Kenngrößen des EKG bzw. dienen der Erkennung begrenzter diagnostischer Aussagen z.B. in US 4,930,075 (Auswertung des ST-Segments zur Feststellung von Ischämien), US 5,025,794 (Methode der bidirektionalen Filterung zur Erkennung von Spätpotentialen), US 5,355,891 (Automatische Signalmittelung durch Schlagtriggerung zur Erkennung von Spätpotentialen), US 5,341,811 (HP-Filterung von mindestens zwei Kanälen, gewichtete Mittelung, Einsatz adaptiver Filter zur Gleichtaktunterdrückung, Spätpotentialerkennung) oder DE 43 04 269 (Auswertung der ST-Strecke zur Bewertung akuter ischämischer Schädigungen).

Die ermittelten Signalkenngrößen werden direkt zusammen mit dem Signalverlauf des EKG auf dem Papierstreifen ausgedruckt oder angezeigt. Zur Ausgabe diagnostischer Hinweise werden in einem mehr oder weniger komplizierten und verzweigten Entscheidungsbaum die einzelnen ermittelten Signalkenngrößen miteinander zu sinnvollen diagnostischen Hinweisen verknüpft. Dies erfolgt beispielsweise durch die Computer-EKG-Geräten zugrundeliegenden Programme. Solche Entscheidungsbäume können beispielsweise folgende Form haben: "Wenn Parameter 1 in Verbindung mit Parameter 3 und/oder Parameter 4 auftritt und in der medizinischen Ableitung a gleichzeitig Bedingung 1 wirksam ist, kann daraus auf die diagnostische Aussage xyz geschlossen werden". In dieser Weise kann für jede bekannte Diagnose ein Entscheidungsbaum auf der Grundlage einzelner, aus dem EKG in seinen Ableitungen ermittelter Signalkenngrößen aufgebaut werden. Dieses Verfahren ist aufgrund der Vielzahl der Einflußgrößen und Parameter außerordentlich aufwendig und setzt umfangreiche kardiologische Kenntnisse bzw. Erfahrungen voraus. Änderungen oder Verbesserungen der Verfahren zur Ermittlung einzelner Parameter, Beeinflussung von empirisch bestimmten Schwellwerten oder neue medizinische Erkenntnisse erfordern teilweise aufwendige Programmänderungen und Funktionstests und sind daher mit hohen Kosten verbunden, bzw. erfordern neue EKG-Geräte mit den überarbeiteten Programmen. Das Patent US 5,355,892 beschreibt daher ein EKG-System mit portablen Speichermedien (Diskettenlaufwerk) zur Speicherung sowohl von EKG und Patienteninformationen, z.B. für Krankenhaus-Informationssysteme als auch zum Nachladen oder Aktualisieren von Algorithmen zur EKG-Auswertung.

In US 5,437,278 wird ein medizinisches Diagnosesystem beschrieben, bei dem digitalisierte medizinische Daten über den Zustand eines Patienten mit in einem Speicher abgelegten, zu einem früheren Zeitpunkt bestimmten, ebenfalls digitalisierten medizinischen Daten verglichen werden. Aus dem Vergleich wird eine Diagnose bezüglich des Patienten abgeleitet.

Der Erfindung liegt die Problemstellung zugrunde, eine von nicht endgültig gesicherten medizinischen Schlußfolgerungen unabhängige und in automatisierter Form mögliche Auswertung periodischer oder quasiperiodischer Signale durch verbesserte Signalvergleiche zu ermöglichen.

Zur Lösung dieses Problems ist erfindungsgemäß das Verfahren der eingangs erwähnten Art dadurch gekennzeichnet, daß die Perioden der gemessenen Signale auf eine vorbestimmte Periodendauer normiert werden und daß die mit einer bestimmten Abtastfrequenz digitalisierten Werte eines Abschnitts der gemessenen und auf die vorbestimmte Periodendauer normierten Signale mit für dieselbe Abtastfrequenz gebildeten Werten eines entsprechenden Abschnitts von in einer Datenbank abgespeicherten und auf dieselbe vorbestimmte Periodendauer normierten Signalen verglichen werden.

Die erfindungsgemäße Auswertung von gemessenen Signalen zur Erzielung diagnostischer Informationen geschieht somit ausschließlich durch einen Signalvergleich mit in der Datenbank abgespeicherten Signalmustern.

Zur Verbesserung der Vergleichbarkeit der gemessenen Signale mit entsprechenden, in einer Datenbank abgespeicherten Signale werden erfindungsgemäß die Vergleichssignale der Datenbank auf eine vorbestimmte Periodendauer normiert und mit einer vorbestimmten Abtastfrequenz digitalisiert. In entsprechender Weise werden die gemessenen Signale auf dieselbe vorbestimmte Periodendauer normiert und mit derselben Abtastfrequenz digitalisiert. Auf diese Weise ist insbesondere erstmalig der Vergleich von entsprechenden Signalen verschiedener Patienten möglich.

Für mehrkanalige Messungen, wie sie beispielsweise beim EKG oder EEG vorkommen, wird zweckmäßigerweise die Auswertung für einzelne Sensorkanäle mit abgespeicherten Signalabschnitten der entsprechenden oder zumindest vergleichbaren Sensorkanäle vorgenommen.

Der Vergleich des aktuell gemessenen Signalmusters mit den in Datenbanken abgelegten Signalen erfolgt vorzugsweise durch die Berechnung eines Korrelationskoeffizienten für jeden Abschnitt der gemessenen Signale mit den Signalen aller oder ausgewählter, in der Datenbank abgespeicherter Signalmuster, und zwar an einer Stelle oder an mehreren Stellen, wobei der Korrelationskoeffizient als Maß für die Ähnlichkeit der verglichenen Signale verwendet wird. Hierdurch wird nur ein Teil der in dem gemessenen Signalmuster enthaltenen diagnostischen Informationen genutzt, dafür jedoch ein einfaches und schnelles Verfahren verwendet, das die Durchführung der benötigten zahlreichen Vergleiche ermöglicht. Werden mehrere Korrelationskoeffizienten bestimmt, wird als Maß für die Ähnlichkeit vorzugsweise das Maximum der Korrelationskoeffizienten verwendet.

Zur Durchführung eines Vergleichs über eine Korrelationsfunktion werden die zu vergleichenden Meßdaten gegeneinander verschoben, um so die Korrelationsfunktion in an sich bekannter Weise zu bilden.

Es ist denkbar, die in der Datenbank abgespeicherten Signalmuster, die alle mit derselben Abtastfrequenz digitalisiert worden sind, für den jeweils durchzuführenden Vergleich ebenfalls auf eine normierte Periodendauer umzurechnen. Vorteilhafter ist es jedoch, die in der Datenbank abgespeicherten Signalmuster bereits als auf die bestimmte Periodendauer normierte und entsprechend digitalisierte Daten abzulegen.

Das erfindungsgemäße Verfahren bietet die Möglichkeit, aus dem Signalvergleich auf einen medizinischen Befund zu schließen, indem den in der Datenbank abgespeicherten Signalen medizinische Befunde zugeordnet sind und nach einer Vielzahl vorgenommener Vergleiche aus einer Häufung von Übereinstimmungen mit abgespeicherten Signalen mit einem bestimmten medizinischen Befund eine Wahrscheinlichkeit für das Vorliegen des bestimmten medizinischen Befunds bezüglich der gemessenen Signale hergeleitet wird.

**Ausführungsbeispiel:**

Die Erfindung soll nachfolgend an einem Ausführungsbeispiel näher erläutert werden. In den dazugehörigen Zeichnungen zeigen

Fig. 1 - Schematische Darstellung der Verfahrensschritte am Beispiel des EKG

Fig. 2 - Darstellung eines Schlages einer Ableitung eines zu vergleichenden EKG vor der Normierung

Fig. 3 -    Darstellung eines Schlages der Ableitung eines zu vergleichenden EKG nach der Normierung

Fig. 4 -    Darstellung der Korrelationsfunktion zweier gut korrelierender Ableitungen von EKG verschiedener Patienten

Fig. 5 -    Darstellung der Korrelationsfunktion zweier schlecht korrelierender Ableitungen von EKG verschiedener Patienten.

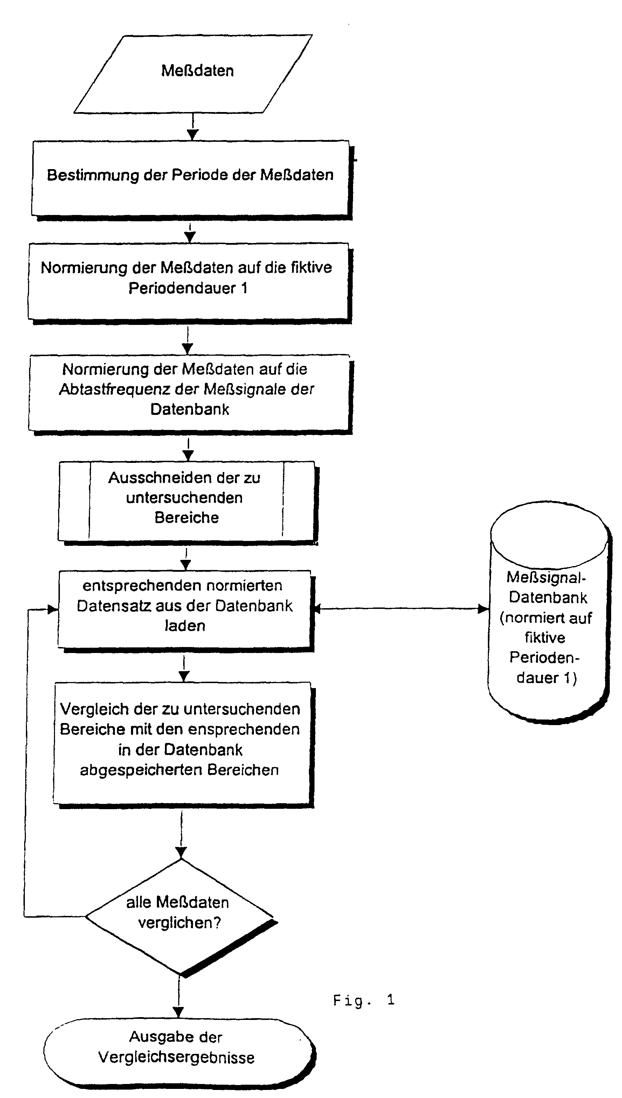

Im nachfolgenden Ausführungsbeispiel werden die einzelnen Verfahrensschritte am Beispiel des Elektrokardiogramm dargestellt (Fig. 1). Ziel eines Vergleiches für EKG-Signale ist es, für jede Ableitung eines gemessenen EKG die oder diejenigen vergleichbaren Ableitungen von EKG in einer EKG-Datenbank zu finden, die in Bezug auf ihre Signalmuster die größtmögliche Übereinstimmung besitzen. Dies erfolgt nach einer Normierung der EKG-Signale mit Hilfe der Berechnung der Korrelationsfunktion, die ein Maß für die Übereinstimmung der Signalmuster zweier Signalausschnitte liefert.

Da jedes EKG unabhängig vom grundlegenden Signalmuster einen individuellen Rhythmus aufweist bzw. sogar innerhalb eines EKG starke Schwankungen des Herzrhythmus auftreten können, ist ein direkter Mustervergleich über die Berechnung der Korrelationsfunktion nicht möglich. Selbst bei identischem Signalmuster jedoch unterschiedlicher Herzrate der zu vergleichenden Ableitungen führt die direkte Berechnung der Korrelationsfunktion zu unterschiedlichen Ergebnissen. Um dennoch einen Vergleich zu ermöglichen, werden die im Signalmuster des EKG enthaltenen Informationen von den im Signalrhythmus enthaltenen getrennt. Dadurch wird es möglich, durch eine Normierung der Signalmuster der EKG verschiedener Patienten (mit unterschiedlicher Herzrate) z.B. auf eine einheitliche, fiktive Herzrate die EKG hinsichtlich ihres Signalmusters mittels Korrelation miteinander zu vergleichen.

Praktisch geschieht dies durch "Stauchen" oder "Strecken" der Zeitachse vergleichbarer Signalabschnitte der Ableitungen der Datenbank-EKG als auch der gemessenen EKG auf eine gleiche, fiktive Länge.

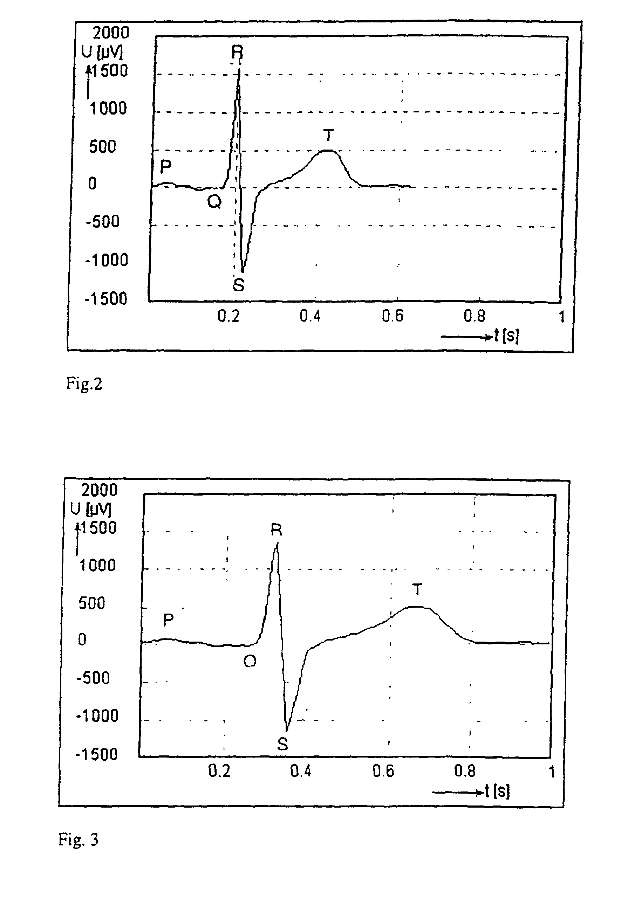

Geht man beispielsweise von einem Schlag als vergleichbaren Signalabschnitt aus (Fig. 2 und Fig. 3), werden die RR-Abstände der zu vergleichenden Ableitungen z.B. auf die Einheitslänge 1 gestaucht oder gestreckt. Durch die vorgegebene Anzahl der Abtastwerte der Ableitung ändert sich bei dieser Anpassung der Zeitachse jedoch dessen Abtastfrequenz. Da die Berechnung der Korrelationsfunktion gleiche Abtastfrequenzen der zu vergleichenden Signalabschnitte voraussetzt, ist eine Neuberechnung der Abtastwerte (Resampling) der gestauchten oder gestreckten Ableitung des gemessenen EKG z.B. durch lineare Interpolation notwendig.

Diese Verfahrensweise führt dazu, daß auch Ableitungen mit z.B. individuell unterschiedlicher Herzfrequenz (unterschiedlichen RR-Abstand) aber mit gleichem Signalmuster gleiche Ergebnisse bei der Berechnung der Korrelationsfunktion ergeben.

Die Korrelationsfunktionen werden für jede Ableitung des Referenz-EKG mit den entsprechenden Ableitungen jedes Datenbank-EKG z.B. nach Gleichung (1) und (2) berechnet.

$$K = K\,(kT_a) \qquad l \le k \le N \tag{1}$$

$$K = \frac{1}{N} \frac{\displaystyle\sum_{n=1}^{N} x_n y_n - \sum_{n=1}^{N} x_n \sum_{n=1}^{N} y_n}{\sqrt{\dfrac{1}{N}\left[\displaystyle\sum_{n=1}^{N} x_n^2 - \left(\sum_{n=1}^{N} x_n\right)^2\right]\left[\displaystyle\sum_{n=1}^{N} y_n^2 - \left(\sum_{n=1}^{N} y_n\right)^2\right]}} \tag{2}$$

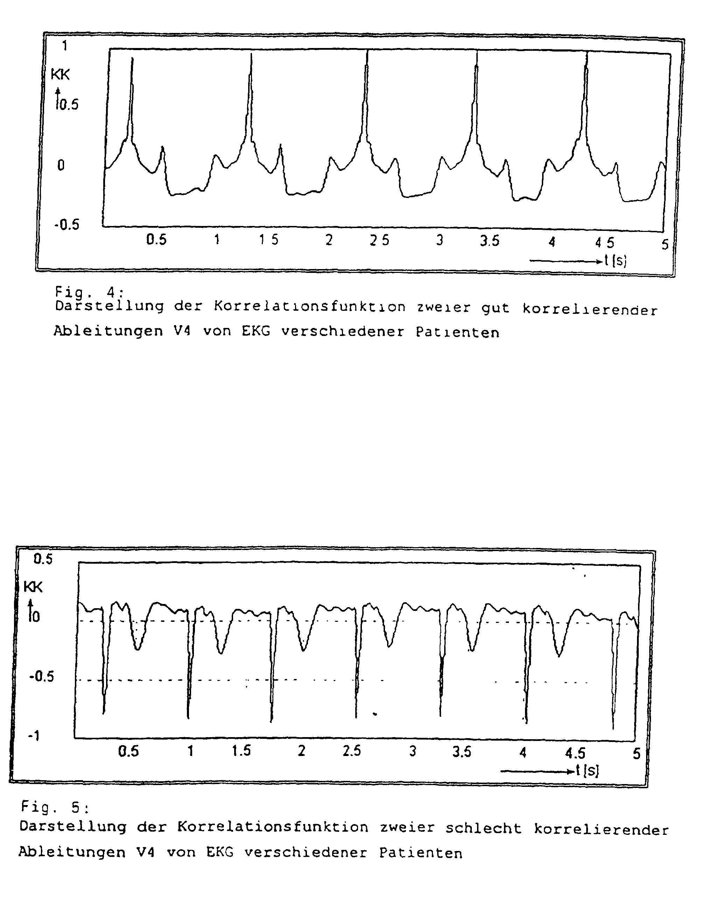

Variable $X_n$ und $Y_n$ kennzeichnen die diskreten Datensätze, die dem auszuwertenden Referenz-EKG X und dem Datenbank-EKG Y entnommen wurden. Die Anzahl Punkte N, an denen die Funktion gebildet werden kann, ergibt sich aus der Länge der zu vergleichenden Signalabschnitte. Für den Signalmusterausschnitt jeder Ableitung des zu vergleichenden EKG wird z.B. eine Periode des EKG-Signals verwendet. Für die Vergleichbarkeit von EKG, die mehrere Ableitungen enthalten, ist es erforderlich, daß die verwendeten Zeitabschnitte jeder Ableitung zum gleichen Zeitpunkt innerhalb des EKG ausgeschnitten werden. Die ausgeschnittene Periode enthält beispielsweise die Signalabschnitte P-Welle, QRS-Komplex und T-Welle. Dieses Signalmuster wird nach Normierung des Zeitmaßstabes mit mindestens einer Schlagperiode der Datenbank-EKG verglichen. Entsprechend dem periodischen Verhalten des Datenbank-EKG ist die Korrelationsfunktion wieder eine periodische Funktion. Die Abb. 4 zeigt den Verlauf der Korrelationsfunktion bei gut übereinstimmenden Signalmustern der korrelierten EKG-Ableitungen. Deutlich sind periodisch wiederkehrende Maxima an den Stellen der größten Übereinstimung der Signalmuster mit einem Amplitudenwert nahe dem Wert 1 ausgeprägt. Abb. 5 zeigt hingegen die Korrelationsfunktion bei weniger gut übereinstimmenden Signalmustern. Die Amplituden der positiven Maxima der Korrelationsfunktion liegen hier unter dem Wert 0.5.

Nach der eingangs formulierten Zielstellung werden die gut übereinstimmenden Signalmuster gesucht. Aus diesem Grunde werden nur die positiven Maximalwerte $M_i$ der Korrelationsfunktion bestimmt. Die Suche nach diesen Maxima erfolgt durch Auswertung der Amplituden unter gleichzeitiger Beachtung der Periodizität des Signals. In Abhängigkeit von den Unterschieden der einzelnen EKG-Schläge einer Ableitung untereinander (Schlagvariation) weichen die Amplituden der periodischen Maxima $M_i$ von einander ab.

Deshalb wird in einem weiteren Schritt entsprechend der Gleichung (3) das absolute Maximum M aus den periodischen Maxima $M_1$ gesucht.

$$ M \ = \ \overset{k}{\underset{i=1}{max}} \left( M_i \right) \qquad\qquad (3) $$

Die so erhaltene Größe M ist ein Maß für die Übereinstimmung der miteinander verglichenen Ableitungen.

Je größer die Ähnlichkeit der Signalmuster, desto größer ist der Wert von M. Bei vollständiger Übereinstimmung der Muster ist M = 1. Im Ergebnis dieser Rechnungen entsteht eine Tabelle (Tabelle 1), deren Spaltenelemente für jede Ableitung den absoluten Maximalwert M der jeweiligen Korrelationsfunktion nach Gleichung (1) beinhaltet. Die Zeilen dieser Tabelle werden durch die für den Vergleich verwendeten Datenbank-EKG gebildet. Zur Kennzeichnung der Datenbank-EKG dient eine EKG-Nummer.

Tabelle 1:

| EKG Nr. | V1 | V2 | V3 | V4 | V5 | V6 | VX | VY | VZ |
|---------|--------|--------|--------|--------|--------|--------|--------|--------|--------|
| 1 | 0.9344 | 0.7158 | 0.4716 | 0.2156 | 0.6730 | 0.8551 | 0.8770 | 0.6391 | 0.8739 |
| 2 | 0.9353 | 0.9137 | 0.8456 | 0.4659 | 0.3440 | 0.6359 | 0.6288 | 0.4124 | 0.9115 |
| 3 | 0.9296 | 0.9026 | 0.5757 | 0.2566 | 0.7614 | 0.8955 | 0.8869 | 0.5512 | 0.8500 |
| 4 | 0.5581 | 0.3083 | 0.1820 | 0.2385 | 0.8575 | 0.9194 | 0.9036 | 0.4272 | 0.3108 |
| 5 | 0.8924 | 0.6752 | 0.4599 | 0.3456 | 0.5817 | 0.8003 | 0.8309 | 0.4432 | 0.7508 |
| 6 | 0.9248 | 0.8974 | 0.5654 | 0.2641 | 0.7069 | 0.8335 | 0.9018 | 0.5178 | 0.8711 |
| 7 | 0.8833 | 0.8781 | 0.7909 | 0.5580 | 0.5524 | 0.8212 | 0.8196 | 0.5462 | 0.7597 |
| 8 | 0.4268 | 0.4237 | 0.4057 | 0.4549 | 0.5289 | 0.8297 | 0.7654 | 0.7184 | 0.3415 |
| 9 | 0.8841 | 0.9335 | 0.6990 | 0.4231 | 0.4224 | 0.6012 | 0.5294 | 0.8438 | 0.9500 |
| 10 | 0.3402 | 0.3667 | 0.2604 | 0.4037 | 0.6145 | 0.3932 | 0.4683 | 0.4891 | 0.7384 |
| Beispiel zur Darstellung der Korrelationsergebnisse (Vergleichs-EKG-Nr. 281) | | | | | | | | | |

Tabelle 1 zeigt die Ergebnisse des Mustervergleichs für die ersten 10 Datenbank-EKG sowie für die Ableitungen V1-V6 und die Frank-Ableitungen Vx, Vy, Vz. Für das Datenbank-EKG 1 ist daraus abzulesen, daß die Ableitung V1 mit der Ableitung V1 des Vergleichs-EKG mit der maximalen Korrelation $M_{V1} = 0{,}93444$, übereinstimmt, die Ableitung V4 jedoch nur mit $M_{V4} = 0{,}21569$.

Es ist für den medizinischen Fachmann klar, daß regelmäßig in Ruhe gemessene EKG-Signale zu vergleichen sind. Unter einer Belastung des Patienten aufgenommene EKG-Signale erfahren eine Veränderung der Signalform, die hinsichtlich der Systole weitgehend unverändert bleibt, hinsichtlich der Diastole jedoch wegen der höheren Herzfrequenz deutlich verkürzt wird. Sollen Belastungs-EKGs in den Vergleich einbezogen werden, muß die entsprechende Signalveränderung für diesen Patienten durch die Belastung gegenüber dem Ruhe-EKG in der Auswertung berücksichtigt werden.

**Patentansprüche**

1. Verfahren zur Auswertung von gemessenen periodischen oder quasiperiodischen Signalen medizinischer Sensorsysteme durch Digitalisierung der Signale und Vergleich von Signalabschnitten der gemessenen Signale mit abgespeicherten vergleichbaren Signalabschnitten, **dadurch gekennzeichnet, daß** die Perioden der gemessenen Signale auf eine vorbestimmte Periodendauer normiert werden und daß die mit einer bestimmten Abtastfrequenz digitalisierten Werte eines Abschnitts der gemessenen und auf die vorbestimmte Periodendauer normierten Signale mit für dieselbe Abtastfrequenz gebildeten Werten eines entsprechenden Abschnitts von in einer Datenbank abgespeicherten und auf dieselbe vorbestimmte Periodendauer normierten Signalen verglichen werden.

2. Verfahren nach Anspruch 1, **dadurch gekennzeichnet, daß** Signalabschnitte verschiedener Patienten miteinander verglichen werden.

3. Verfahren nach Anspruch 1 oder 2, **dadurch gekennzeichnet, daß** für mehrkanalige Messungen Signale einzelner Sensorkanäle mit abgespeicherten Signalen vergleichbarer Sensorkanäle verglichen werden.

4. Verfahren nach einem der Ansprüche 1 bis 3, **dadurch gekennzeichnet, daß** für jeden Abschnitt der gemessenen Signale mit den Signalen aller oder ausgewählter in der Datenbank abgespeicherter Signalmuster ein Korrelationskoeffizient an einer Stelle oder mehreren Stellen berechnet und als Maß für die Ähnlichkeit der verglichenen Signale verwendet wird.

5. Verfahren nach Anspruch 4, **dadurch gekennzeichnet, daß** aus ermittelten Korrelationskoeffizienten eine Korrelationsfunktion gebildet wird.

6. Verfahren nach Anspruch 4 oder 5, **dadurch gekennzeichnet, daß** bei der Berechnung mehrerer Korrelationskoeffizienten deren Maximum als Maß für die Ähnlichkeit der verglichenen Signale verwendet wird.

7. Verfahren nach einem der Ansprüche 1 bis 6, **dadurch gekennzeichnet, daß** die in der Datenbank abgespeicherten Signalmuster bereits als auf die bestimmte Periodendauer normierte und entsprechend digitalisierte Daten abgelegt werden.

8. Verfahren nach einem der Ansprüche 1 bis 7, **dadurch gekennzeichnet, daß** bei EKG-Signalen die Periodendauer im Signalmuster entsprechend dem RR-Abstand und/oder dem Mittelwert mehrerer RR-Abstände gewählt wird.

9. Verfahren nach einem der Ansprüche 1 bis 8, **dadurch gekennzeichnet, daß** bei EKG-Signalen mit feststellbarer R-Zackenposition der Korrelationskoeffizient nur an der Stelle ermittelt wird, an der die R-Zacken der zu vergleichenden Bereiche übereinander liegen.

10. Verfahren nach einem der Ansprüche 1 bis 9, **dadurch gekennzeichnet, daß** bei EKG-Signalen ohne feststellbare R-Zackenposition die Korrelationsfunktion über mindestens eine Periode des EKG-Signals berechnet wird.

11. Verfahren nach einem der Ansprüche 1 bis 10, **dadurch gekennzeichnet, daß** den in der Datenbank abgespeicherten Signalen medizinische Befunde zugeordnet sind und daß nach einer Vielzahl vorgenommener Vergleiche aus einer Häufung von Übereinstimmungen mit abgespeicherten Signalen mit einem bestimmten medizinischen Befund eine Wahrscheinlichkeit für das Vorliegen des bestimmten medizinischen Befunds bezüglich der gemessenen Signale hergeleitet wird.

12. Verfahren nach Anspruch 11, **dadurch gekennzeichnet, daß** die Länge der Abschnitte so gewählt wird, daß sie nur einen Teil einer diagnostischen Information enthalten.

**Claims**

1. A process for evaluating measured periodic or quasiperiodic signals of medical sensor systems by digitization of the signals and comparison of segments of the measured signals with stored comparable signal segments, **characterised in that** the periods of the measured signals are normalized to a predetermined cycle duration and in that the values, digitized with a particular sampling frequency, of a segment of the signals that have been measured and normalized to the predetermined cycle duration are compared with values, produced for the same sampling frequency, of a corresponding segment of signals that have been stored in a database and normalized to the same predetermined cycle duration.

**2.** Process according to Claim 1, **characterised in that** signal segments of various patients are compared with one another.

**3.** Process according to Claim 1 or 2, **characterised in that** for multi-channel measurements signals of individual sensor channels are compared with stored signals of comparable sensor channels.

**4.** Process according to one of Claims 1 to 3, **characterised in that** for each segment of the measured signals a coefficient of correlation with the signals of all or selected signal patterns stored in the database is calculated at one point or at several points and is used as a measure of the similarity of the compared signals.

**5.** Process according to Claim 4, **characterised in that** a correlation function is formed from ascertained correlation coefficients.

**6.** Process according to Claim 4 or 5, **characterised in that** in connection with the calculation of several correlation coefficients the maximum thereof is used as a measure of the similarity of the compared signals.

**7.** Process according to one of Claims 1 to 6, **characterised in that** the signal patterns stored in the database are already saved in the form of data that have been normalized to the particular cycle duration and digitized appropriately.

**8.** Process according to one of Claims 1 to 7, **characterised in that** in the case of ECG signals the cycle duration in the signal pattern is chosen so as to correspond to the R-R interval and/or to the mean value of several R-R intervals.

**9.** Process according to one of Claims 1 to 8, **characterised in that** in the case of ECG signals with determinable R-wave position the correlation coefficient is ascertained only at the point where the R waves of the regions to be compared are superposed.

**10.** Process according to one of Claims 1 to 9, **characterised in that** in the case of ECG signals without determinable R-wave position the correlation function is calculated over at least one period of the ECG signal.

**11.** Process according to one of Claims 1 to 10, **characterised in that** medical findings are assigned to the signals stored in the database and in that after a plurality of comparisons have been performed a probability for the existence of the particular medical finding with respect to the measured signals is derived from an accumulation of instances of coincidence with stored signals associated with a particular medical finding.

**12.** Process according to Claim 11, **characterised in that** the length of the segments is so chosen that said segments contain only a fraction of diagnostic information.

**Revendications**

**1.** Procédé pour traiter des signaux périodiques ou quasipériodiques mesurés fournis par des systèmes capteurs médicaux, en numérisant les signaux et en comparant des segments de signal des signaux mesurés avec des segments de signal comparables issus d'une mémoire, **caractérisé en ce que** les périodes des signaux mesurés sont normées à une durée de période prédéterminée et en ce que les valeurs numérisées avec une fréquence d'échantillonnage déterminée d'un segment des signaux mesurés et normés sur la durée de période prédéterminée sont comparées avec des valeurs constituées pour la même fréquence d'échantillonnage, d'un segment correspondant de signaux normés à la même durée de période prédéterminée, stockées dans une banque de données.

**2.** Procédé selon la revendication 1, **caractérisé en ce que** les segments de signal de différents patients sont comparés entre eux.

**3.** Procédé selon la revendication 1 ou 2, **caractérisé en ce que** pour des mesures multi-canaux, des signaux fournis par des canaux de capteur individuels sont comparés avec des signaux stockés, de canaux de capteur comparables.

**4.** Procédé selon l'une des revendications 1 à 3, **caractérisé en ce que** pour chaque segment des signaux mesurés on calcule un coefficient de corrélation en un endroit ou plusieurs endroits avec les signaux de tous ou certains choisis des modèles de signaux stockés dans la banque de données, et on utilise ce coefficient de corrélation comme mesure pour la similitude des signaux comparés.

**5.** Procédé selon la revendication 4, **caractérisé en ce que** l'on constitue une fonction de corrélation à partir de coefficients de corrélation obtenus.

**6.** Procédé selon la revendication 4 ou 5, **caractérisé en ce que**, plusieurs coefficients de corrélation étant calculés, on utilise leur maximum comme mesure pour la similitude des signaux comparés.

**7.** Procédé selon l'une des revendications 1 à 6, **caractérisé en ce que** les modèles de signaux stockés dans la banque de données sont déjà déposés en tant que données normées à la durée de période déterminée, et numérisées de façon correspondante.

**8.** Procédé selon l'une des revendications 1 à 7, **caractérisé en ce que** concernant des signaux d'électrocardiogramme, la durée de période dans le modèle de signal est choisie pour correspondre à la distance RR et/ou à la moyenne de plusieurs distances RR.

**9.** Procédé selon l'une des revendications 1 à 8, **caractérisé en ce que** concernant des signaux d'électrocardiogramme où la dent R a une position déterminable, le coefficient de corrélation n'est établi qu'en la position où les dents R des zones à comparer se superposent.

**10.** Procédé selon l'une des revendications 1 à 9, **caractérisé en ce que** concernant des signaux d'électrocardiogramme sans position de dent R déterminable, la fonction de corrélation est calculée sur au moins une période du signal d'électrocardiogramme.

**11.** Procédé selon l'une quelconque des revendications 1 à 10, **caractérisé en ce qu'**aux signaux stockés dans la banque de données sont associés des rapports médicaux, et en ce qu'après avoir effectué de multiples comparaisons, on déduit d'une accumulation de coïncidences avec des signaux stockés associés à un rapport médical déterminé une vraisemblance que le rapport médical déterminé s'applique en ce qui concerne les signaux mesurés.

**12.** Procédé selon la revendication 11, **caractérisé en ce qu'**on choisit la longueur des segments de façon qu'ils ne contiennent qu'une partie d'une information diagnostique.

Meßdaten

Bestimmung der Periode der Meßdaten

Normierung der Meßdaten auf die fiktive Periodendauer 1

Normierung der Meßdaten auf die Abtastfrequenz der Meßsignale der Datenbank

Ausschneiden der zu untersuchenden Bereiche

Meßsignal-Datenbank (normiert auf fiktive Perioden-dauer 1)

entsprechenden normierten Datensatz aus der Datenbank laden

Vergleich der zu untersuchenden Bereiche mit den ensprechenden in der Datenbank abgespeicherten Bereichen

alle Meßdaten verglichen?

Fig. 1

Ausgabe der Vergleichsergebnisse

9

Fig.2

Fig. 3

Fig. 4:
Darstellung der Korrelationsfunktion zweier gut korrelierender
Ableitungen V4 von EKG verschiedener Patienten

Fig. 5:
Darstellung der Korrelationsfunktion zweier schlecht korrelierender
Ableitungen V4 von EKG verschiedener Patienten